**Europäisches Patentamt**

**European Patent Office**

**Office européen des brevets**

(19)

(11)  **EP 1 026 615 A1**

(12)  **EUROPÄISCHE PATENTANMELDUNG**

(43)  Veröffentlichungstag:
09.08.2000  Patentblatt 2000/32

(51)  Int Cl.⁷: $G06F\ 19/00$, $G06F\ 17/18$

(21)  Anmeldenummer: 99102275.7

(22)  Anmeldetag: 05.02.1999

(84)  Benannte Vertragsstaaten:
AT BE CH CY DE DK ES FI FR GB GR IE IT LI LU
MC NL PT SE
Benannte Erstreckungsstaaten:
AL LT LV MK RO SI

(71)  Anmelder: **Liebel, Franz-Peter, Dr.**
**D-33619 Bielefeld (DE)**

(72)  Erfinder: **Liebel, Franz-Peter, Dr.**
**D-33619 Bielefeld (DE)**

(54)  **Verfahren zur Berechnung von Wahrscheinlichkeiten pathophysiologischer Zustände als Zwischenergebnisse der Diagnosefindung**

(57)  Es wird ein Verfahren vorgestellt, das für eine vorgegebene Symptommenge die Zutreffenswahrscheinlichkeiten der kausal zugeordneten Ereignisse zu berechnen vermag. Dies entspricht - im Fall einer Anwendung in der Medizin - der Berechnung von Wahrscheinlichkeiten pathophysiologischer Zustände.

Für die Berechnungen werden Ereignisse mit sicher bestimmter, sowie Ereignisse mit unbekannter Zutreffenswahrscheinlichkeit eingesetzt. Für jede der unbekannten Zutreffenswahrscheinlichkeiten stellt man eine Bestimmungsgleichung auf und erhält so bei x Unbekannten ein System von x nicht-linearen Gleichungen.

Mit der Einführung von Voraussetzungen, die im hauptsächlich vorgesehenen Anwendungsbereich der medizinischen Diagnostik gut zu erfüllen sind, können die in den Gleichungen erscheinenden bedingten Wahrscheinlichkeiten so vereinfacht werden, daß die jeweiligen Bedingungen im allgemeinen nur noch ein Ereignis aufweisen. Dadurch wird die Erstellung von Stichproben zur Ermittlung der entsprechenden Zahlenwerte problemlos.

EP 1 026 615 A1

**Beschreibung**

A ) Verwendbarkeit der Erfindung

Es ist problematisch, die im Verlauf eines medizinischen Diagnoseprozesses angehäuften Daten und Meßwerte zu überblicken und zu werten. Eine entscheidende Hilfe kann ein Verfahren geben, das für die angefallenem Meßwerte kausal zugeordnte pathophysiologische Zustände bestimmt, indem es deren Zutreffenswahrscheinlichkeiten berechnet.

Ein solches Verfahren ist dann vergleichbar einem Zusammenfassen und Bündeln einzelner Daten zur Gewinnung einer Meßgröße oder einer Aussage für einen "übergeordneten" pathologischen Zustands des Gewebes.

Im Hinblick auf die oft nur vage zielgerichteten Laboruntersuchungen, und im Hinblick auf die Notwendigkeit gezielt vorgenommener Untersuchungen mit bildgebenden medizinischen Geräten, kann das vorgestellte Verfahren integraler Bestandteil werden von z.B. Laborautomaten, Computertomographen u.ä.

B) Stand der Technik und Vorteile des Verfahrens

Zu Anspruch 1

Durch die in Anspruch 1 angegebene Gleichung vermeidet man a-priori-Wahrscheinlichkeiten (z.B. p(H), p($K_1$),... ), deren Zahlenwerte nur schwer zu ermitteln sind.

Für eine Wahrscheinlichkeit der Form p(F | K J H) ist es wesentlich, daß die Operationen "Interpolation" und "Zerlegung bzgl. der Ereignisse vor dem Bedingungsstrich" in genau dieser Reihenfolge ausgeführt werden.

Zu Anspruch 2

Da die im Bedingungsverbund einer bedingten Wahrscheinlichkeit stehenden '-gekennzeichneten Ereignisse Separationseigenschaft besitzen ( vergleiche Abschnitt C), Erläuterung zum Linearen Interpolationssatz ), ist eine lineare Interpolation nur auszuführen, wenn die Voraussetzung gem. Anspruch 2 erfüllt ist.

Zu Anspruch 3

Es hat sich gezeigt, daß bei nicht-linearer Interpolation nur eine Gleichbehandlung aller '-gekennzeichneten Ereignisse die nötige Einfachheit herbeiführt.

Interpolationen mit verschiedenen $V_{E_i}$, oder gar unstetige Interpolationen, sind aufgrund des beträchtlichen Aufwands nicht geeignet.

Interpoliert man gleichbleibend mit maximalem $V_{E_i}$, so ist man, bei Berücksichtigung der Ereignisse des Bedingungsverbundes, nächstmöglich an der linearen Interpolation.

Zu Anspruch 4 und 5

Mit einer einzigen "wirklichen" Voraussetzung, nämlich der Unabhängigkeit der Ereignisse in

$$\bigcup_i (URS(F_i) \cup INH(F_i)),$$

erreicht man die Lösung der Aufgabe.

Die Kritik, dadurch sei das Postulat der Unabhängigkeit lediglich von der Folgen-Ebene auf die Hypothesen-Ebene verlagert worden, trifft nicht zu, da die Folgen wegen gemeinsamer Ursachen per definitionem abhängig sind; eine solche Abhängigkeit liegt für die Ereignisse der H/K-Ebene i.a. nicht vor.

Zu Anspruch 6

Es gelingt die Zerlegung von bedingten Wahrscheinlichkeiten, auch wenn sowohl vor als auch nach dem Bedingungsstrich ein unter Umständen umfangreicher Er-eignisverbund steht.

Man erreicht durch die Verkleinerungen schließlich bedingte Wahrscheinlichkeiten, die vor und nach dem Bedingungsstrich nur noch ein einzelnes Ereignis aufweisen. Da die Wahrscheinlichkeiten zudem von der Form $p$

*(Folge | Ursache)* sind, wird die Ermittlung der zugehörigen Zahlenwerte aus Stichproben problemlos.

Zu Anspruch 7

Durch die gleichzeitige Erfassung aller unbekannten Zutreffenswahrscheinlichkeiten in einem Gleichungssystem ist die Schwierigkeit bewältigt, daß sich die '-gekennzeichneten Ereignisse einer kausalen Struktur wechselseitig beeinflussen.

C) Darstellung der Lösung und Beispiel

1. Einleitung

Es hat sich als schwierig erwiesen hat, eine brauchbare sogenannte Bewertungsfunktion zu konzipieren. Eine solche Bewertungsfunktion ist ein Verfahren, das anhand einer vorgegebenen Ereignismenge die Wahrscheinlichkeiten kausal verknüpfter Ereignisse, insbesondere von Ursachen ermittelt. Dabei meint "Ermittlung einer unbekannten Wahrscheinlichkeit" die konkrete Berechnung eines Zahlenwertes mit Methoden der Diskreten Stochastik.
Es hat sich zudem gezeigt, daß das kleine Gebiet der Diskreten Stochastik für ein derartiges Unterfangen nicht die benötigten Werkzeuge bereithält. Einen ersten Schritt zur Fortsetzung der klassischen Stochastik durch Einbeziehung unsicher bestimmter Ereignisse liefert die lineare Interpolation

$$p(H|E') = p(H|E)p(E|E') + p(H|\bar{E})p(\bar{E}|E'). \qquad (1.1)$$

Im weiteren wird sich recht eindrucksvoll ergeben, daß die Interpolation der Glg.1.1 ein Sonderfall des Allgemeinen Interpolationssatzes ist, wenn dort gilt: $(E_1...E_h E'_{h+1}...E'_k) := E'$ (vergl. Abschnitt 3., Glg.3.1).

2. Kausalstruktur

Die Ereignisse werden zweckmäßigerweise in einem Kausalnetz geordnet, dem die Bezeichnung L-Netz gegeben wurde. Diese L-Netz ist im wesentlichen dadurch charakterisiert, daß die Ereignisse durch zunächst beliebig viele gerichtete Kausalverweise verknüpft sind, wobei zugelassen ist, daß auf die Kausalverbindungen Inhibitoren einwirken, und daß die Inhibitionsmechanismen wiederum durch hemmende Ereignisse verstärkt oder gemindert werden können.
Eine inhibitorische Beeinflussung einer beliebigen Kausalverbindung $U_3 \rightarrow H$ im nachfolgenden Beispiel der Abb.2.1 bedeutet, daß Ereignisse bestehen, die eine Erzeugung von H durch $U_3$ hemmend beeinflussen. Ist $U_3$.H die Kurzbezeichnung des kausalen Vorgangs "$U_3$ erzeugt H", so wird der hemmende Einfluß ausgedrückt durch:

$$p(U_3.H \,|U_3) \geq p(U_3.H| \ U_3 \ I_1). \qquad (2.1)$$

Die so gebildete Grundstruktur erhält schließlich durch die Voraussetzungen des Abschnitts 4. die endgültige Gestalt.
Das nachfolgende Beispiel eines L-Netzes enthält negierte und unnegierte Ereignisse, sowie durch □? gekennzeichnete Netzknoten mit unbekannter Zutreffenswahrscheinlichkeit.

Ursachen-Ebene $\quad$ $U_1$ $\square$ $\quad$ $U_2$ $\square$? $\quad$ $U_3$ $\square$? $\quad$ $I_1$ $\square$ $\quad$ $I_2$ $\square$

Hypothesen-Ebene $\quad$ $K_1$ $\square$? $\quad$ $J_1$ $\square$ $\quad\quad$ $H$ $\square$? $\quad$ $K_2$ $\square$ $\quad$ $K_3$ $\square$? $\quad$ $\overline{J_2}$ $\square$

Folgen-Ebene $\quad$ $F_1$ $\square$ $\quad$ $F_2$ $\square$ $\quad$ $F_3$ $\square$ $\quad$ $\overline{F}_4$ $\square$ $\quad$ $F_5$ $\square$

Abb.2.1:

Beispiel eines L-Netzes. Die gewählten Bezeichnungen der Netz-knoten sind willkürlich. Das Netz kann im Bedarfsfall über die □? – Ereignisse beliebig fortgesetzt werden.

Es werden folgende Bezeichnungen festgelegt, die für den beliebig gewählten Netzknoten H formuliert sind:

URS(H) $\quad$ Menge der unmittelbaren Ursachen von H.

FOL(H) $\quad$ Menge der unmittelbaren Folgen von H.

DIFF(H) $\quad$ Menge der von H verschiedenen Netzknoten, die unmittelbare Ursache für Elemente aus FOL(H) sind. (DIFF(H) umfaßt diejenigen Elemente, die z.B. bei Anwendungen in der Medizin differentialdiagnostisch zu H in Betracht zu ziehen sind.)

INH(Z) $\quad$ Menge der Netzknoten, die über Schaltstellen die auf einen beliebigen Netzknoten Z hinführenden Kausalverbindungen inhibitorisch beeinflussen.

WERT(H) $\quad$ Menge aller Netzknoten mit Einfluß auf die Zutreffenswahrscheinlichkeit von H (Wertungsumgebung von H).

Der Menge WERT(H) wird zugewiesen:

$$WERT(H) := URS(H) \cup FOL(H) \cup DIFF(H) \cup INH(H) \cup \bigcup_{Z \in FOL(H)} INH(Z) \; . \; (2.2)$$

Der aus den Elementen der Menge WERT(H) gebildete Verbund (synonym: Produkt, Durchschnitt) wird mit W(H) bezeichnet. Die Ereignisse darin sind dann je nach Zutreffenswahrscheinlichkeit negiert, unnegiert oder '-gekennzeichnet. Mit W(H) wird definiert:

*Definition ('-Kennung)*

*Für die zugrundegelegte Kausalstruktur und ein darin enthaltenes, beliebiges Element H ist H' : = W(H).*

Für das Beispiel der Abb.2.1 erhält man so als Zutreffenswahrscheinlichkeit des Ereignisses H :

$$p(H \mid H') = p(H \mid W(H))$$

$$= p(H \mid U_1 \ U_2' \ U_3' \ F_1 \ F_2 \ F_3 \ \overline{F}_4 \ F_5 \ K_1' \ K_2 \ K_3' \ I_1 \ I_2 \ J_1 \ \overline{J}_2 ). \qquad (2.3)$$

### 3. Interpolationsformeln

Benötigt werden Formeln zur Berechnung von Wahrscheinlichkeiten der Form $p(H \mid E_1...E_h E'_{h+1}...E'_k)$, wobei die Ereignisse $E_1, ... , E_h$ sicher bestimmt, d.h. negiert oder unnegiert vorliegen, während die Ereignisse $E_i$', i = h+1, ..., k, eine (bekannte oder unbekannte) Zutreffenswahrscheinlichkeit $0 < p(E_i \mid E_i') < 1$ besitzen und demzufolge eine '-Kennung tragen.

In den Formeln werden Ausdrücke der Form $p(E_i \mid V_{E'_i})$, i = h + 1, ... , k verwendet, wobei $V_{E'_i}$ einen auch leer zugelassenen Verbund bezeichnet, der aus dem Verbund $(E_1...E_h E'_{h+1}...E'_k)$ dadurch entsteht, daß man $E'_i$ entfernt, sowie beliebige und beliebig viele weitere Elemente.

Für die Interpolationsfunktionen zur Berechnung von $p(H \mid E_1...E_h E'_{h+1}...E'_k)$ wird gefordert, daß die Interpolation für alle i = h+1, ..., k die nachfolgenden drei Interpolationspunkte erfüllt:

### 1. Interpolationspunkt:

$$p(H \mid E_1...E_h E'_{h+1}...E'_i...E'_k) = p(H \mid E_1...E_h E'_{h+1}...\bar{E}_i...E'_k) \text{ für } p(E_i \mid E'_i) = 0.$$

### 2. Interpolationspunkt:

$$p(H \mid E_1...E_h E'_{h+1}...E'_i...E'_k) = p(H \mid E_1...E_h E'_{h+1}...E_i...E'_k) \text{ für } p(E_i \mid E'_i) = 1.$$

### 3. Interpolationspunkt:

$$p(H \mid E_1...E_h E'_{h+1}...E'_i...E'_k) = p(H \mid E_1...E_h E'_{h+1}...E'_{i-1} E'_{i+1}...E'_k)$$

für

$$p(E_i \mid E'_i) = p(E_i \mid V_{E'_i})$$

bei beliebig gewähltem $V_{E'_{h+1}}$.

(Die Bedeutung des 3. Interpolationspunktes besteht darin, daß genau dann, wenn $p(E_i \mid E'_i)$ den Wert $p(E_i \mid V_{E'_i})$ besitzt, kein updating von p(H | H') bzgl $E'_i$ stattfindet.)

*Allgemeiner Interpolationssatz*

*Für $p(H \mid E_1...E_h E'_{h+1}...E'_k)$ gelte:*

- *$E_1, ... , E_h$ sind negiert oder unnegiert (beliebig aber fest).*
- *$E'_{h+1}, ....,E'_k$ besitzen $0 < p(E_i \mid E'_i) <1$, i = h+1, ... , k.*
- *Es sei $q_i \in \{0,1\}$, i = h+1, ... , k mit $E^{(q_i)} = \bar{E}_i$ für $q_i = 0$ und $E^{(q_i)} = E_i$ für $q_i = 1$.*
- *$V_{E'_i}$ ist ein beliebiger, auch leer zugelassener teilverbund von*

$$(E_1...E_h E'_{h+1}...E'_{i-1}E'_{i+1}...E'_k),\ i = h + 1, ... , k.$$

*Dann genügen folgende Gleichungen den geforderten drei Interpolationspunkten:*

$$p(H \mid E_1...E_h E'_{h+1}...E'_k) = \frac{\displaystyle\sum_{q_{h+1},...,q_k=0,1} p(HE_1...E_h E_{h+1}^{(q_{h+1})}...E_k^{(q_k)}) \prod_{i=h+1}^{k} \frac{p(E_i^{(q_i)} \mid E'_i)}{p(E_i^{(q_i)} \mid V_{E'_i})}}{\displaystyle\sum_{q_{h+1},...,q_k=0,1} p(E_1...E_h E_{h+1}^{(q_{h+1})}...E_k^{(q_k)}) \prod_{i=h+1}^{k} \frac{p(E_i^{(q_i)} \mid E'_i)}{p(E_i^{(q_i)} \mid V_{E'_i})}} , (3.1)$$

$$E_i^{(0)} = \bar{E}_i\ und\ E_i^{(1)} = E_i,\ i = h+1, ... , k.$$

Beweis: Elementar.

Die Anwendung der Glg.3.1 oder eines davon abgeleiteten Satzes ist ein updating-Verfahren.

Das updating so gewählt, daß $p(H \mid H')$ infolge $E_i'$ genau dann nicht verändert wird, wenn $p(E_i \mid E_i')$ den Wert besitzt, den es durch das Vorliegen der ohnehin für $p(H \mid H')$ verwendeten Ereignisse $(E_1...E_h E'_{h+1}...E'_{i-1}E'_{i+1}...E'_k)$ erreichen kann, wenn also $p(E_i \mid E_i') = p(E_i \mid E_1...E_h E'_{h+1}...E'_{i-1}E'_{i+1}...E'_k)$ gilt.

Für das updating im L-Netz wird somit i.a. das maximale $V_{E'_i}$ eingesetzt, d.h. es wird $V_{E'_i} := (E_1...E_h E'_{h+1}...E'_{i-1}E'_{i+1}...E'_k)$ verwendet.

Am Beispiel $p(U_2 \mid U_1 I_1 I_2 H' U_3')$ wird die Handhabung der Glg.3.1 demonstriert. Hierzu werden $V_{H'}$ und $V_{U'_3}$ größtmöglich gewählt zu $V_{H'} := (U_1 I_1 I_2 U_3')$ bzw. $V_{U'_3} := (U_1 I_1 I_2 H')$. (Hier wäre jeweils auch jeder Teilverbund möglich, einschließlich des leeren Verbunds.)

(Wählt man etwa $V_{U'_3} := (U_1 I_1 I_2)$, so erscheint wegen $p(U_3 \mid U_1 I_1 I_2) = p(U_3)$ eine der a-priori-Wahrscheinlichkeiten, deren Zahlenwerte schwer zu ermitteln sind.) Es ergibt sich gem. Glg.3.1:

$$p(U_2 \mid U_1\ I_1\ I_2\ H'\ U_3') \qquad\qquad (3.2)$$

$$= \frac{p(U_2 U_1 I_1 I_2 H U_3)\dfrac{p(H\mid H')}{p(H\mid U_1 I_1 I_2 U_3')}\dfrac{p(U_3\mid U_3')}{p(U_3\mid U_1 I_1 I_2 H')}+}{p(U_1 I_1 I_2 H U_3)\dfrac{p(H\mid H')}{p(H\mid U_1 I_1 I_2 U_3')}\dfrac{p(U_3\mid U_3')}{p(U_3\mid U_1 I_1 I_2 H')}+}\ \cdots$$

$$\cdots\ \frac{p(U_2 U_1 I_1 I_2 H\overline{U_3})\dfrac{p(H\mid H')}{p(H\mid U_1 I_1 I_2 U_3')}\dfrac{p(\overline{U_3}\mid U_3')}{p(\overline{U_3}\mid U_1 I_1 I_2 H')}+}{p(U_1 I_1 I_2 H\overline{U_3})\dfrac{p(H\mid H')}{p(H\mid U_1 I_1 I_2 U_3')}\dfrac{p(\overline{U_3}\mid U_3')}{p(\overline{U_3}\mid U_1 I_1 I_2 H')}+}\ \cdots$$

$$\cdots\ \frac{p(U_2 U_1 I_1 I_2 \overline{H}U_3)\dfrac{p(\overline{H}\mid H')}{p(\overline{H}\mid U_1 I_1 I_2 U_3')}\dfrac{p(U_3\mid U_3')}{p(U_3\mid U_1 I_1 I_2 H')}+}{p(U_1 I_1 I_2 \overline{H}U_3)\dfrac{p(\overline{H}\mid H')}{p(\overline{H}\mid U_1 I_1 I_2 U_3')}\dfrac{p(U_3\mid U_3')}{p(U_3\mid U_1 I_1 I_2 H')}+}\ \cdots$$

$$\cdots\ \frac{p(U_2 U_1 I_1 I_2 \overline{H U_3})\dfrac{p(\overline{H}\mid H')}{p(\overline{H}\mid U_1 I_1 I_2 U_3')}\dfrac{p(\overline{U_3}\mid U_3')}{p(\overline{U_3}\mid U_1 I_1 I_2 H')}}{p(U_1 I_1 I_2 \overline{H U_3})\dfrac{p(\overline{H}\mid H')}{p(\overline{H}\mid U_1 I_1 I_2 U_3')}\dfrac{p(\overline{U_3}\mid U_3')}{p(\overline{U_3}\mid U_1 I_1 I_2 H')}}\ .$$

An dem Beispiel ist auch leicht die Erfüllung der Interpolationspunkte zu sehen.

*Spezielle Interpolationssätze*

*L-Satz*

*Mit $V_{E'_i} := \varnothing$ für alle i = h+1, ..., k ergibt sich aus Glg.3.1 ein einfacherer Interpolationssatz, der infolge seiner Entstehungsgeschichte die Bezeichnung L-Satz führt.*

*Linearer Interpolationssatz*

*Mit maximalem $V_{E'_i}$ oder $V_{E'_i} := (E_1...E_h)$ ergibt sich aus Glg.3.1 die "Lineare Interpolation" (linear in den $p(E_i \mid E'_i)$), falls zusätzlich a) oder b) gilt:*

*a) $(E'_{h+2}...E'_k) = \varnothing$.*

*b) $\{E_{h+1},..., E_k\}$ sind unter der Bedingung $(E_1.....E_h W(E_{h+1})^*.....W(E_k)^*)$ stochastisch unabhängig, wobei $W(E_i)^* := (W(E_i)$ abzgl. $\{H\} \cup \{E_1,..., E_h\})$.*

*Mit den Voraussetzungen a) oder b) erhält man aus Glg.3.1:*

$$p(H \mid E_1 \ldots E_h E'_{h+1} \ldots E'_k) = \sum_{q_{h+1},\ldots,q_k=0,1} p(H \mid E_1 \ldots E_h E^{(q_{h+1})}_{h+1} \ldots E^{(q_k)}_k) \prod_{i=h+1}^{k} p(E^{(q_i)}_i \mid E'_i),$$

$$E^{(0)}_i = \overline{E_i} \quad und \quad E^{(1)}_i = E_i, \; i = h+1, \ldots, k. \tag{3.3}$$

Erläuterung zu b):

Gegeben sei die Struktur:

(Nur eingezeichnete Ereignisse sind existent.)

<u>Vorbemerkung</u>

Es ist $p(F_1 \mid K_1' K_2') \neq p(F_1 \mid K_1' K_2' F_2) \neq p(F_1 \mid K_1' K_2' \overline{F}_2)$. Jede dieser Wahrscheinlichkeiten kann sich auf eine andere Kausalstruktur, nämlich eine Struktur ohne $F_2$, mit $F_2$ und mit $\overline{F}_2$ beziehen.

Es gilt jedoch $p(F_1 \mid K_1' K_2') = p(F_1 \mid K_1' K_2' F_2)$, wenn vorausgesetzt wird, daß nur eine Kausalstruktur zugrunde liegt. Diese Kausalstruktur ist bestimmend für die $p(K_1 \mid K_1')$ und $p(K_2 \mid K_2')$, die zwar unbekannt, bei vorgegebener Struktur aber fest sind. Bezeichnung: $K_1'$, $K_2'$ <u>separieren</u> $F_1$ von $F_2$.

Für die angegebene Struktur wird nun gezeigt, daß die Anwendung der Glg.3.1 auf $p(F_1 \mid W(F_1))$ keine lineare Interpolation ergibt:

$$
\begin{aligned}
p(F_1 \mid W(F_1)) \quad &= p(F_1 \mid K_1' K_2') \\[4pt]
&\underset{\text{(Separationseigenschaft)}}{=} p(F_1 \mid K_1' K_2' W(K_1)^* \; W(K_2)^*) \\[4pt]
&= p(F_1 \mid K_1' K_2' F_2) \\[4pt]
&= (\text{Anwendung der Glg.3.1 mit } V_{K_1'} = V_{K_2'} := (F_2')) \\[6pt]
&\underset{\text{(Glg.3.1)}}{=} \frac{p(F_1 F_2 K_1 K_2) \dfrac{p(K_1 \mid K_1') \; p(K_2 \mid K_2')}{p(K_1 \mid F_2) \; p(K_2 \mid F_2)} + \ldots}{p(F_2 K_1 K_2) \dfrac{p(K_1 \mid K_1') \; p(K_2 \mid K_2')}{p(K_1 \mid F_2) \; p(K_2 \mid F_2)} + \ldots} \ldots \\[8pt]
&\neq \quad p(F_1 \mid F_2 K_1 K_2) \, p(K_1 \mid K_1') \, p(K_2 \mid K_2') + \ldots \\[4pt]
&\qquad (\text{wegen } p(K_1 K_2 \mid F_2) \neq p(K_1 \mid F_2) \, p(K_2 \mid F_2)) \\[4pt]
&\underset{\text{(unabhängig)}}{=} p(F_1 \mid K_1 K_2) \, p(K_1 \mid K_1') \, p(K_2 \mid K_2') + \ldots
\end{aligned}
$$

Wegen Nichterfüllung der Ziff. b) liefert Glg.3.1 somit keine linare Interpolation.

Die Formel (3.3) wird am Beispiel $p(H \mid U_1 \, I_1 \, I_2 \, U_2' \, U_3')$ verdeutlicht. Bei stochastischer Unabhängigkeit von $U_2$ und $U_3$ unter der Bedingung $(U_1 \, I_1 \, I_2)$, sowie der Abwesenheit ent-separierender Ereignisse in $U_2'$, $U_3'$, ergibt sich:

$$p(H \mid U_1 \, I_1 \, I_2 \, U_2' \, U_3') =$$

$$p(H \mid U_1\, I_1\, I_2\, U_2\, U_3)\; p(U_2 \mid U_2')\; p(U_3 \mid U_3') +$$

$$p(H \mid U_1\, I_1\, I_2\, U_2\, \overline{U}_3)\; p(U_2 \mid U_2')\; p(\overline{U}_3 \mid U_3') +$$

$$p(H \mid U_1\, I_1\, I_2\, \overline{U}_2\, U_3)\; p(\overline{U}_2 \mid U_2')\; p(U_3 \mid U_3') +$$

$$p(H \mid U_1\, I_1\, I_2\, \overline{U}_2\, \overline{U}_3)\; p(\overline{U}_2 \mid U_2')\; p(\overline{U}_3 \mid U_3'). \tag{3.4}$$

Die Glgn.3.1 und 3.3 sind Interpolationen und im allgemeinen keine Gleichheit. (Kriterien für die Erreichung von Gleichheit vergl. Abschnitt 7.)

4. Voraussetzungen

Die L-Netze werden durch die nachstehenden Voraussetzungen so strukturiert, daß sich die vorgesehenen Berechnungen vereinfachen. Dabei ist zu beachten, daß die zugrunde gelegte Kausalstruktur, d.h. die Verknüpfungen der pathophysiologischen Zustände, solche Voraussetzungen zulassen. Es gelten Bezeichnungen, die für den beliebigen Knoten H am Beispiel der Abb.2. 1 formuliert sind:

U    Verbund der Elemente aus URS(H); U:= $(U_1\, U_2'\, U_3')$.
F    Verbund der Elemente aus FOL(H); F:= $(F_1\, F_2\, F_3\, \overline{F}_4\, F_5)$.
K    Verbund der Elemente aus DIFF(H); K:= $(K_1'\, K_2\, K_3')$.
     ( K erinnert an "konkurrierend zu H".)
I    Verbund der Elemente aus INH(H); I:= $(I_1\, I_2)$.
J    Verbund der Elemente aus INH$(F_1)$,..., INH$(F_5)$; J:= $(J_1\, \overline{J}_2)$.

*Die folgenden, für H formulierten Voraussetzungen I, IIa, IIb, IIc und III, sowie die ergänzenden Voraussetzungen IIa\*, IIb\* und IIc\* gelten entsprechend für jeden nicht-inhibitorischen Netzknoten mit unbekannter Zutreffenswahrscheinlichkeit.*

*Voraussetzung I*

*Die Ereignisse aus F, I und J liegen mit der Wahrscheinlichkeit Null oder Eins vor.*

Erläuterung zu Voraussetzung I

Das gestellte Problem ist die Ermittlung der Wahrscheinlichkeiten hypothetischer Ursachen, also der Wahrscheinlichkeiten für Ereignisse aus der H/K-Ebene, der U-Ebene oder "weiter darüber liegender" Ursachen (vergl. Abb. 2.1). Grundlage für eine solche Ermittlung sind Erkenntnisse über Folge-Ereignisse und Inhibitoren, die zugunsten der Ergebnisgenauigkeit nicht unsicher bestimmt sein sollen.

*Voraussetzung IIa*

*Die Mengen URS$(F_i)$, $F_i$ aus F, sollen sämtliche, diesen Mengen zugehörigen Ereignisse enthalten.*

Erläuterung zu Voraussetzung IIa

Auch bei nicht-computergestützten Verfahren leidet die Diagnose, wenn für einzelne Symptome nicht alle in Frage kommenden Ursachen berücksichtigt sind. Der vornehmliche Grund für die vorliegende Forderung ist jedoch, daß die Ereignisse $F_i$ mit Hilfe einer Bedingung stochastisch unabhängig werden können, wenn diese Bedingung sämtliche Elemente aus URS$(F_i)$ enthält.

*Voraussetzung IIb*

*Die Ereignisse in URS$(F_i)$, $F_i$ aus F, sind selbstständige Ursachen der $F_i$.*

Erläuterung zu Voraussetzung IIb

Die Selbständigkeit von Ursachen ist für den nachfolgend eingeführten A.L-Satz exakt definiert. Anschaulich bedeutet es, daß für einen beliebigen Netzknoten L

- die Ereignisse in URS(L) stochastisch unabhängig sind, und daß
- ein beliebiger L-erzeugender Vorgang nicht durch andere Ursachen oder durch die Inhibitoren eines weiteren L-erzeugenden Vorgangs beeinflußt wird.

Im Fall medizinischer Anwendungen kann die Erfüllung der Voraussetzung IIb angenommen werden, wenn die einzelnen Ursachen als nicht "nahe verwandt" anzusehen sind.

*Voraussetzung IIc*

*Stochast. Unabhängigkeit gilt für die Elemente in URS($F_i$) $\cup$ INH($F_i$), $F_i$ aus F.*

Erläuterung zu Voraussetzung IIc

Für die $F_i$ aus F erreicht man Unabhängigkeit mittels eines Bedingungsverbundes, der alle Ursachen der $F_i$ enthält, wenn noch zudem alle Elemente in URS($F_i$) $\cup$ INH($F_i$) unabhängig sind.

*Voraussetzung III*

$$p(U\ F\ K\ I\ J\ |\ H) = p(UI|\ H)\ p(F\ K\ J\ |\ H)\ \text{für H und }\bar{H}.$$

Erläuterung zu Voraussetzung III

Die Gleichung der Voraussetzung III ist die mathematische Formulierung der durch H bewirkten Separierung von (U I) und (F K J). Daraus ergibt sich für die Kausalstruktur, daß Elemente aus (U I) nur über H mit den Knoten des "darunter" liegenden Netzes verknüpft sein dürfen.

*Die Voraussetzungen IIb und IIc wurden separat formuliert, da sie im weiteren in diesen Formen verwendet werden. Es besteht jedoch die Entsprechung IIb $\Leftrightarrow$ IIc.*

Ergänzende Voraussetzungen

Die Voraussetzungen IIa, IIb und IIc sind formuliert für URS($F_i$) und INH($F_i$), $F_i$ aus F. Völlig analoge Voraussetzungen können für URS(H) und INH(H) gefordert werden; sie seien mit IIa*, IIb* und IIc* bezeichnet.

5. Berechnung der Zutreffenswahrscheinlichkeiten

Mit den getroffenen Voraussetzungen ergibt sich für p(H | H') = p(H | U F K I J):

$$p(H \mid UFKIJ) = \frac{p(HUFKIJ)}{p(HUFKIJ) + p(\overline{H}UFKIJ)}$$

$$= \frac{1}{1 + \dfrac{p(UFKIJ \mid \overline{H})p(\overline{H})}{p(UFKIJ \mid H)p(H)}}$$

$$=_{(\text{Vorauss.III})} \frac{1}{1 + \dfrac{p(FKJ\overline{H})p(UI \mid \overline{H})}{p(FKJH)p(UI \mid H)}}$$

$$=_{(\text{Vorauss.IIc})} \frac{1}{1 + \dfrac{p(F \mid KJ\overline{H})p(\overline{H} \mid UI)}{p(F \mid KJH)p(H \mid UI)}} \qquad . \qquad\qquad (5.1)$$

Zur weiteren Bearbeitung wird p(F | K J H) linear interpoliert (die maßgebende Ziffer b) ist erfüllt, da alle ent-separierenden $F_i$ vor dem Bedingungsstrich stehen). Dasselbe Ergebnis liefert der L-Satz, 5-fach angewendet auf die rechte Seite von: p($F_1$...$F_5$ | K J H) = p($F_1$ | $F_2$...$F_5$ K J H) p($F_2$ | $F_3$...$F_5$ K J H) ·...· p($F_5$ | K J H). Mit Glg.3.3 (oder dem L-Satz) erhält man bei n '-Elementen $K_1'$, ..., $K_n'$:

$$\mathrm{p}(F \mid K_1{}^{\text{‘}} \dots K_n{}^{\text{‘}} \, J\,H) = \sum_{q_1,\dots,q_n=0,1} p(F \mid K_1^{(q_1)}\dots K_n^{(q_n)}JH)\,p(K_1^{(q_1)} \mid K_1')\cdot\dots\cdot p(K_n^{(q_n)} \mid K_n')\,.$$

Da der Bedingungsverbund sämtliche Ursachen der $F_i$ aus F enthält, und da die Elemente in INH($F_1$) $\cup$ ...$\cup$ INH($F_5$) stochastisch unabhängig sind, folgt:

$$p(F \mid K_1^{(q_1)}\dots K_n^{(q_n)}JH) = \prod_i p(F_i \mid K_1^{(q_1)}\dots K_n^{(q_n)}JH)\,, \; F_i \text{ aus F }. \qquad (5.1a)$$

Die Wahrscheinlichkeiten der Form p(Folge | Ursachen $\wedge$ Inhibitoren) können unter Nutzung der Selbstständigkeitseigenschaft der Elemente in URS($F_i$) und URS(H) weiter vereinfacht werden. Sei hierzu:

L    Beliebiges Ereignis.
A    Beliebig gewählter, aber feststehender Verbund aus unnegierten Ereignissen von URS(L) mit A:= ($A_1$... $A_k$).
X    Beliebiger Verbund mit negierten oder unnegierten Ereignissen aus URS(L) \ {Elemente in A}.
A.L    Kausaler Vorgang "A erzeugt L" . Ist A ein Verbund mit A:= ($A_1$...$A_k$), so bedeutet A.L: " $A_1$ erzeugt L" $\cup$ ... $\cup$ "$A_k$ erzeugt L".
D    Beliebiger Verbund mit Ereignissen, die für $A_1$.L,..., $A_k$.L Inhibitoren sind, oder die Inhibition von $A_1$.L,..., $A_k$.L verstärken oder abschwächen.

*Definition (Selbständigkeit)*

*A heißt selbständige Ursache von L, falls A für alle X die nachfolgenden Voraussetzungen 1/A.L bis 4/A.L erfüllt:*

*Voraussetzung 1/A.L: p((A.L) (X.L) | AX) = p(A.L | A X) p(X.L | A X).*
*Voraussetzung 2/A.L:      p(A.L | A X) = p(A.L | A ).*
*Voraussetzung 3/A.L:        p(X.L | A X) = p(X.L | $\bar{A}_1$...$\bar{A}_k$X).*
*Voraussetzung 4/A.L:          p(X | A) = p(X | $\bar{A}_1$...$\bar{A}_k$).*

Damit ergibt sich:

*A.L-Satz und A.L-Korollar 1*

*Unter der Voraussetzung, daß A eine selbständige Ursache von L ist, gilt mit A:= (A₁...Aₖ):*

$$A.L\text{-Satz: } p(A.L \mid A) = \frac{p(L|A)\text{-}p(L|\bar{A}_1...\bar{A}_k)}{p(\bar{L}|\bar{A}_1...\bar{A}_k)}. \qquad (5.2)$$

$$A.L\text{-Korollar 1: } p(A.L \mid A\,D) = \frac{p(L|AD)\text{-}p(L|\bar{A}_1...\bar{A}_kD)}{p(\bar{L}|\bar{A}_1...\bar{A}_kD)}. \qquad (5.3)$$

Mit dem A.L-Satz bzw. dem A.L-Korollar 1 lassen sich Wahrscheinlichkeiten der Form *p(Folge | Ursachen)* entscheidend vereinfachen.

Dies wird exemplarisch für p(H | UI) gezeigt. Da die Ereignisse in (U I) die Voraussetzung b) des Linearen Interpolationssatzes erfüllen, wird Glg.3.3 angewendet, wodurch Wahrscheinlichkeiten der Form p(H | U₁ ... U₃ I₁ ... I₂) entstehen (vergl. Glg.3.4). Wegen der Voraussetzungen IIa* und IIb* folgt für diese Ausdrücke:

$$p(H \mid U_1\,U_2\,U_3\,I_1\,I_2) \;=\; 1 - \prod_{i=1}^{3}(1 - p(U_i.H \mid U_1...U_3I_1...I_2)) \qquad (5.4)$$

Bezeichnet $I_{U_i.H}$ einen Teilverbund von (I₁...I₂), ausschließlich bestehend aus Inhibitoren fur den im Index stehenden Kausalvorgang $U_i$.H, so gilt unter Anwendung des A.L-Korollars 1 für alle i:

$$p(U_i.H \mid U_1...U_3\,I_1\,...\,I_2) \;=\; p(U_i.H \mid U_i I_{U_i.H})$$

$$= \; \frac{p(H \mid U_i I_{U_i.H}) - p(H \mid \overline{U}_i I_{U_i.H})}{p(\overline{H} \mid \overline{U}_i I_{U_i.H})}. \qquad (5.5)$$

(Bei $\bar{U}_i$ ist zu beachten, daß $p(U_i.H \mid \bar{U}_i) = 0$ gilt.)

Eine Vereinfachung der rechten Seite von Glg.5.5, so daß der Bedingungsverbund zusätzlich zur Ursache $U_i$ nur noch einen Inhibitor aufweist, ist im Abschnitt 8. angegeben.

6. Berechnung eines Kausalnetzes am Beispiel der Abb.2.1

Eine Kausalstruktur wie in Abb.2.1 wird über die '-gekennzeichneten Ereignisse der H/K-Ebene fortgesetzt. Dieses kann so erfolgen, daß für jedes dieser Ereignisse modulartig ein L-Netz erstellt wird, wie es mit dem Beispiel der Abb.2.1 für H demonstriert ist. Die einzelnen Module sind übersichtlicher und bilden zusammengesetzt die Gesamt-Kausalstruktur.

Für das Berechnungsbeispiel seien R₁ und R₃ die Repräsentanten solcher modulartigen Fortsetzungen, z. B. R₁ aus dem L-Netz-Modul für K₁ und R₃ aus dem L-Netz-Modul für K₃, wobei exemplarisch R₁ ∈ URS(K₁) und R₃ ∈ URS(K₃) gewählt werden kann.

Schritt 1

Für die erste Verdachtshypothese H wird ein L-Netz erstellt, das die Ereignismengen URS(H), FOL(H), DIFF(H), INH(H) und

$$\bigcup_{Z \in FOL(H)} INH(Z)$$

enthält, wobei Vollzähligkeit für die Menge DIFF(H) in jedem Fall (Voraussetzung IIa) , und für die Menge URS(H) im Bedarfsfall (Voraussetzung IIa*) zu fordern ist.

Insgesamt sind für die Strukturierung des L-Netzes die Voraussetzungen I, IIa, IIc und III, sowie IIa* und IIc* maßgebend.

Es interessieren die Zutreffenswahrscheinlichkeiten der '-gekennzeichneten Ereignisse in der H/K-Ebene. Für jedes dieser Ereignisse wird nach denselben Grundsätzen wie für H ein L-Netz-Modul erstellt.

Vorteil gegenüber anderen Systemen

Nicht zufriedenstellend gelöst war bisher das Problem, daß sich die Zutreffenswahrscheinlichkeiten der '-gekennzeichneten Ereignisse eines Kausalnetzes wechselseitig beeinflussen. Durch die gleichzeitige Erfassung aller unbekannten Zutreffenswahrscheinlichkeiten in einem Gleichungssystem (wie es nachfolgend vorgestellt wird) ist diese Schwierigkeit behoben.

Schritt 2

Für jedes '-gekennzeichnete Ereignis des Kausalnetzes wird unter Verwendung der Glg.2.2 eine sogenannte Wertungsgleichung erstellt. Man erhält das folgende Gleichungssystem der Glgn. 6.1 bis 6.5:

$$
\begin{aligned}
p(H \mid H') \quad &= \ p(H \mid W(H)) \\
&= \ p(H \mid U \ F \ K \ I \ J) \\
&= \ (\ \text{ident.} \ \text{Glg.2.3}). 
\end{aligned}
\tag{6.1}
$$

$$
\begin{aligned}
p(K_1 \mid K_1') &= \ p(K_1 \mid W(K_1)) \\
&= \ p(K_1 \mid F_1 \ F_2 \ H' \ J_1 \ R_1).
\end{aligned}
\tag{6.2}
$$

$$
\begin{aligned}
p(K_3 \mid K_3') &= \ p(K_3 \mid W(K_3)) \\
&= \ p(K_3 \mid F_5 \ H' \ \overline{J}_2 \ R_3).
\end{aligned}
\tag{6.3}
$$

$$
\begin{aligned}
p(U_2 \mid U_2') &= \ p(U_2 \mid W(U_2)) \\
&= \ p(U_2 \mid H' \ U_1 \ U_3' \ I_1 \ I_2).
\end{aligned}
\tag{6.4}
$$

$$
\begin{aligned}
p(U_3 \mid U_3') &= \ p(U_3 \mid W(U_3)) \\
&= \ p(U_3 \mid H' \ U_1 \ U_2' \ I_1 \ I_2).
\end{aligned}
\tag{6.5}
$$

Man wird in praxi den Aufwand für $p(U_2 \mid U_2')$ und $p(U_3 \ U_3')$, oder gar für weiter "darüber" liegende Ereignisse, möglichst gering halten.

Es sollte jedoch $(U\ I) \neq \emptyset$ sein, um in Glg.5.1 die schwer zu ermittelnde a-priori-Wahrscheinlichkeit $p(H)$ zu vermeiden. Auch aus diesem Grund interpoliert man die Glgn.6.4 und 6.5 mit *maximalem* $V_{E'_i}$, so daß $p(E_i \mid V_{E'_i}) \neq p$

($E_i$) gilt.

Schritt 3

Man erhält bei Anwendung der Glg.3.1 bzw. der Glg.5.1 das Gleichungssystem der Glgn.6.1a bis 6.5a wie folgt:

$$p(H \mid H') =_{(Glg.5.1)} \cfrac{1}{1 + \cfrac{p(F_1...F_5 \mid K_1' K_2 K_3' J_1 \bar{J}_2 H) p(\bar{H} \mid UI)}{p(F_1...F_5 \mid K_1' K_2 K_3' J_1 \bar{J}_2 H) p(p(H \mid UI)}} . \qquad (6.1a)$$

In Glg.6.1a ist:

$$p(F_1...F_5 \mid K_1' K_2 K_3' J_1 \bar{J}_2 H)$$

$$= \sum_{q_1, q_3 = 0,1} p(F_1...F_5 \mid K_1^{(q_1)} K_2 K_3^{(q_3)} J_1 \bar{J}_2 H) p(K_1^{(q_1)} \mid K_1') p(K_3^{(q_3)} \mid K_3') .$$

In dieser Gleichung wiederum ist

$$p(F_1...F_5 \mid K_1^{(q_1)} K_2 K_3^{(q_3)} J_1 \bar{J}_2 H) = \prod_{i=1}^{5} p(F_i \mid K_1^{(q_1)} K_2 K_3^{(q_3)} J_1 \bar{J}_2 H),$$

wobei gilt:

$$p(F_1 \mid K_1^{(q_1)} K_2 K_3^{(q_3)} J_1 \bar{J}_2 H) = p(F_1 \mid K_1^{(q_1)} H),$$

$$p(F_2 \mid K_1^{(q_1)} K_2 K_3^{(q_3)} J_1 \bar{J}_2 H) = p(F_2 \mid K_1^{(q_1)} J_1 H),$$

$$p(F_3 \mid K_1^{(q_1)} K_2 K_3^{(q_3)} J_1 \bar{J}_2 H) = p(F_3 \mid K_2 H),$$

$$p(\bar{F}_4 \mid K_1^{(q_1)} K_2 K_3^{(q_3)} J_1 \bar{J}_2 H) = p(\bar{F}_4 \mid K_2 H),$$

$$p(F_5 \mid K_1^{(q_1)} K_2 K_3^{(q_3)} J_1 \bar{J}_2 H) = p(F_5 \mid K_3^{(q_3)} \bar{J}_2 H).$$

Zudem ist in Glg.6.1a:

$$p(H \mid U I) = p(H \mid U_1 U_2' U_3' I_1 I_2)$$
$$= (ident. Glg.3.4).$$

$$p(K_1 \mid K_1') =_{(Glg.5.1)} \cfrac{1}{1 + \cfrac{p(F_1 F_2 \mid H' J_1 \bar{K}_1) p(\bar{K}_1 \mid R_1)}{p(F_1 F_2 \mid H' J_1 K_1) p(K_1 \mid R_1)}} . \qquad (6.2a)$$

(Analog Glg.6.1a; ebenso die übrigen Ereignisse der H/K-Ebene.)

$$p(K_3 \mid K_3') =_{(Glg.5.1)} \frac{1}{1+\dfrac{p(F_5|H'\bar{J}_2\bar{K}_3)p(\bar{K}_3|R_3)}{p(F_5|H'\bar{J}_2K_3)p(K_3|R_3)}}. \tag{6.3a}$$

$$p(U_2 \mid U_2') =_{(Glg.3.1)} (\text{ident.Glg.3.2}). \tag{6.4a}$$

$$p(U_3 \mid U_3') =_{(Glg.3.1)} (\text{ wie Glg.6.4a; } U_2, U_3 \text{ vertauscht }). \tag{6.5a}$$

### Schritt 4

Die nicht-bedingten Wahrscheinlichkeiten der Glgn.6.4a und 6.5a (vergl. dazu die ausgeschriebene Form der Glg.3.2) werden mittels einfacher Umformungen, z.B.

$$p(U_2 \, U_1 \, I_1 \, I_2 \, H \, U_3) =_{(IIc^*)} p(H \mid U_1 \, U_2 \, U_3 \, I_1 \, I_2) \, p(U_1) \, p(U_2) \, p(U_3) \, P(I_1) \, p(I_2),$$

in die für den nachstehenden Schritt 5 erforderliche Form gebracht.

### Schritt 5

Die bedingte Wahrscheinlichkeit eines Ereignisses, wobei der Bedingungsverbund alle Ursachen des betreffenden Ereignisses enthält, wird mit Glg.5.4 und dem A.L-Satz bzw. dem A.L-Korollar 1 berechnet. Beispiel:

$$p(H \mid U_1 \, U_2 \, U_3 \, I_1 \, I_2) =_{(Glg.5.4)} 1 - (1 - p(U_1.H \mid U_1))$$
$$\cdot (1 - p(U_2.H \mid U_2))$$
$$\cdot (1 - p(U_3.H \mid U_3)),$$

wobei exemplarisch gilt:

$$p(U_1.H \mid U_1) =_{(Glg.5.2)} \frac{p(H|U_1)\text{-}p(H|\bar{U}_1)}{p(\bar{H}|\bar{U}_1)}.$$

Mit Schritt 5 ist erreicht, daß nur solche bedingten Wahrscheinlichkeiten erforderlich sind, die in der Bedingung eine einzelne Ursache und (wegen Anhang 2) höchstens einen Inhibitor genau dieser Ursache aufweisen. Die dafür nötigen Stichproben zur Ermittlung der Zahlenwerte sind (da die erzeugende Ursache in der Bedingung steht) vergleichsweise einfach zu gewinnen.

### Schritt 6

Mit den Glgn.6.1 bis 6.5 und den nachfolgenden Berechnungsschritten steht somit ein nicht-lineares Gleichungssystem in den Unbekannten $p(H \mid H')$, $p(K_1 \mid K_1')$, $p(K_3 \mid K_3')$, $p(U_2 \mid U_2')$ und $p(U_3 \mid U_3')$ zur Verfügung, das iterativ mit Hilfe des nicht-linearen Einzel- oder Gesamtschrittverfahrens gelöst wird. Hierbei kann im Startvektor für eine beliebige Unbekannte $p(Y \mid Y')$ derjenige Zahlenwert verwendet werden, den man (z.B. im Fall von Anwendungen in der Medizin) durch eine nicht-computergestützte Diagnostik erhalten würde. Der Startvektor bildet so die erste Näherung für das gestellte Problem.

7. Gleichheit bei linearer Interpolation

Es stellt sich die Frage, unter welchen Umständen für die Interpolation nach dem Linearen Interpolationssatz Gleichheit erreicht werden kann.

Die Problematik wird vereinfachend am Beispiel der Abb.2.1 dargestellt. Hierzu wird p(H | $U_1 I_1 I_2 U_2' U_3'$) gewählt:

$$
p(H \mid U_1\,I_1\,I_2\,U_2`\,U_3`) = \underset{\text{(Erweiterung)}}{} \frac{\displaystyle\sum_{q_2,q_3=0,1} p(HU_1I_1I_2U_2'U_3'U_2^{(q_2)}U_3^{(q_3)})}{\displaystyle\sum_{q_2,q_3=0,1} p(U_1I_1I_2U_2'U_3'U_2^{(q_2)}U_3^{(q_3)})}
$$

$$
= \frac{p(H \mid U_1I_1I_2U_2'U_3'U_2U_3)\,p(U_2U_3 \mid U_1I_1I_2U_2'U_3') + .....}{p(U_2U_3 \mid U_1I_1I_2U_2'U_3') + .....} .....
$$

$$(7.1)$$

Um für die Glg.7.1 die Form des Linearen Interpolationssatzes zu erreichen, muß gelten (bei beliebiger Negierung von $U_2$ und U3):

$$p(H \mid U_1\,I_1\,I_2\,U_2'\,U_3'\,U_2\,U_3) = p(H \mid U_1\,I_1\,I_2\,U_2\,U_3). \tag{7.2}$$

$$p(U_2\,U_3 \mid U_1\,I_1\,I_2\,U_2'\,U_3') = p(U_2 \mid U_2')\,p(U_3 \mid U_3'). \tag{7.3}$$

Unter Beachtung der getroffenen Voraussetzungen und der davon abhängenden strukturellen Zuordnung der Ereignisse, ergibt sich:

1.

Glg.7.2 gilt, falls zu $U_2'$ und $U_3'$ nur Ereignisse gehören, die ausschließlich über $U_2$ und $U_3$ strukturell mit H verbunden sind, oder die bereits im Bedingungsverbund stehen.

2.

Glg.7.3 gilt, falls zu $U_2'$ und $U_3'$ keine Ereignisse gehören, so daß die vorausgesetzte Unabhängigkeit der Elemente in (U I) aufgehoben wird; eine Aufhebung der stochastischen Unabhängigkeit zweier Ereignisse wird u.a. durch eine gemeinsame, mit p = 1 vorliegende Folge bewirkt.

8. Ergänzung zu Glg.5.5

*Sei H ein beliebiger Netzknoten, $U_1$ sei eine beliebige Ursache von H, und $I_1,...,I_x$ seien die Inhibitoren von $U_1.H$. Zusätzlich zu den Voraussetzungen des Abschn.4. gelte: Unter der Bedingung $U_1$ sind die Inhibitoren $I_1,..., I_x$ selbständige Ursachen des Ereignisses $(\overline{U_1.H})$ . Dann gilt:*

$$p(U_1.H \mid U_1\,I_1\,...\,I_x) = \frac{p(U_1.H|U_1I_1)}{p(U_1.H|U_1)} \cdot ..... \cdot \frac{p(U_1.H|U_1I_x)}{p(U_1.H|U_1)} \cdot p(U_1.H|U_1). \tag{8.1}$$

Beweis:

Das A.L-Korollar 2 sagt aus (vergl. (2), S.121), daß zwei stochastisch unabhängige Ereignisse A und B, die selbständige Ursachen eines Ereignisses L sind, unter der Bedingung $\overline{L}$ unabhängig bleiben. Damit sind die $I_1,..., I_x$ unter der Bedingung $(\overline{U_1.H})$, also auch unter der Bedingung $(U_1\,(U_1.H))$ stochastisch unabhängig. Es folgt:

$$p(U_1.H \mid U_1 I_1 \dots I_x) = \frac{p(I_1 \dots I_x \mid U_1(U_1.H)) \cdot p(U_1(U_1.H))}{p(U_1)p(I_1) \cdot \dots \cdot p(I_x)}$$

$$= \frac{p(I_1 \mid U_1(U_1.H))}{p(I_1)} \cdot \dots \cdot \frac{p(I_x \mid U_1(U_1.H))}{p(I_x)} \cdot p(U_1.H \mid U_1)$$

$$= \frac{p(U_1.H \mid U_1 I_1)}{p(U_1.H \mid U_1)} \cdot \dots \cdot \frac{p(U_1.H \mid U_1 I_x)}{p(U_1.H \mid U_1)} \cdot p(U_1.H \mid U_1), \quad \text{q.e.d.}$$

Ende.

**Patentansprüche**

1. Verfahren zur Ermittlung von Wahrscheinlichkeiten pathophysiologischer Zustände als Zwischenergebnisse der Diagnosefindung, dadurch gekennzeichnet, daß man für ein beliebiges Ereignis H mit der unbekannten Zutref-fenswahrscheinlichkeit $0 < p(H \mid H') < 1$ die Beziehung

$$p(H \mid U \, F \, K \, I \, J) = \frac{1}{1 + \frac{p(F \mid KJ\overline{H})p(\overline{H} \mid UI)}{p(F \mid KJH)p(H \mid UI)}}$$

anwendet, und daß man dann für $F := (F_1 \, F_2 \, F_3 \dots F_r)$ eine r-fache Anwendung des L-Satzes auf die rechte Seite der Gleichung

$$p(F \mid K \, J \, H) = p(F_1 \mid F_2 \, F_3 \dots F_r \, K \, J \, H) \, p(F_2 \mid F_3 \dots F_r \, K \, J \, H) \cdot \dots \cdot p(F_r \mid K \, J \, H)$$

ausführt, äquivalent einer linearen Interpolation von $p(F \mid K \, J \, H)$.

2. Verfahren zur Ermittlung von Wahrscheinlichkeiten pathophysiologischer Zustände als Zwischenergebnisse der Diagnosefindung, dadurch gekennzeichnet, daß man für ein beliebiges Ereignis H mit der unbekannten Zutref-fenswahrscheinlichkeit $0 < p(H \mid H') < 1$ die Beziehung

$$p(H \mid E_1 \dots E_h E'_{h+1} \dots E'_k) = \sum_{q_{h+1}, \dots, q_k = 0,1} p(H \mid E_1 \dots E_h E_{h+1}^{(q_{h+1})} \dots E_k^{(q_k)}) \prod_{i=h+1}^{k} p(E_i^{(q_i)} \mid E'_i) ,$$

$$E_i^{(0)} = \overline{E}_i \text{ und } E_i^{(1)} = E_i, \, i = h+1, \dots, k.$$

genau dann einsetzt, wenn gilt:
$\{ E_{h+1}, \dots, E_k )$ sind stochastisch unabhängig unter der Bedingung des Ereignisverbundes $(E_1 \dots E_h \, W(E_{h+1})^* \dots W(E_k)^*)$, wobei der Verbund $W(E_i)^*$ für alle i definiert ist als $W(E_i)^* := ( \, W(E_i) \text{ abzgl. } \{H\} \cup \{E_1, \dots, E_h\})$.

3. Verfahren zur Ermittlung von Wahrscheinlichkeiten pathophysiologischer Zustände als Zwischenergebnisse der Diagnosefindung, dadurch gekennzeichnet, daß man für ein beliebiges Ereignis H mit der unbekannten Zutref-fenswahrscheinlichkeit $0 < p(H \mid H') < 1$ im Falle einer nicht-linearen Interpolation die Beziehung

$$p(H \mid E_1...E_h E'_{h+1}...E'_k) =$$

$$\frac{\sum_{q_{h+1},...,q_k=0,1} p(HE_1...E_h E^{(q_{h+1})}_{h+1}...E^{(q_k)}_k) \prod_{i=h+1}^{k} \frac{p(E^{(q_i)}_i \mid E'_i)}{p(E^{(q_i)}_i \mid E_1...E_h E'_{h+1}...E'_{i-1} E'_{i+1}...E'_k)}}{\sum_{q_{h+1},...,q_k=0,1} p(E_1...E_h E^{(q_{h+1})}_{h+1}...E^{(q_k)}_k) \prod_{i=h+1}^{k} \frac{p(E^{(q_i)}_i \mid E'_i)}{p(E^{(q_i)}_i \mid E_1...E_h E'_{h+1}...E'_{i-1} E'_{i+1}...E'_k)}} \,,$$

$$E^{(0)}_i = \bar{E}_i \; und \; E^{(1)}_i = E_i, \; i = h+1, ..., k.$$

einsetzt, daß man für sämtliche nicht-linearen Interpolationen genau diese Beziehung anwendet, und daß dafür der Term $p(E^{(q_i)}_i \mid E_1...E_h E'_{h+1}...E'_{i-1} E'_{i+1}...E'_k)$ in der Formel stets verwendet wird.

4. Verfahren zur Ermittlung von Wahrscheinlichkeiten pathophysiologischer Zustände als Zwischenergebnisse der Diagnosefindung, dadurch gekennzeichnet, daß man für ein beliebiges Ereignis H mit der unbekannten Zutreffenswahrscheinlichkeit $0 < p(H \mid H') < 1$ bei Verwendung der Bezeichnungen

U        Verbund der Ursachen von H,
F        Verbund der Folgen von H,
K        Verbund der Ereignisse, die mit H eine gemeinsame Folge besitzen,
I        Verbund der Inhibitoren von Kausalverbindungen, die zu H führen,
J        Verbund der Inhibitoren von Kausalverbindungen, die zu Folge-Ereignissen von H führen,
URS(H)   Menge der Ursachen von H,
FOL(H)   Menge der Folgen von H,

als Voraussetzungen fordert, daß

- die Ereignisse aus F, I, J mit der Wahrscheinlichkeit Null oder Eins vorliegen,
- (H K), im Bedarfsfall auch U, sämtliche, diesen Verbunden zuzuordnenden Ereignisse enthalten,
- die Ereignisse in (H K), im Bedarfsfall auch die Ereignisse in U, selbständige Ursachen sind,
- die Ereignisse in

$$\bigcup_{F_i \in F} (URS(F_i) \cup INH(F_i)) \,,$$

im Bedarfsfall auch die Ereignisse in URS(H) $\cup$ INH(H), stochastische Unabhängigkeit aufweisen, und daß
- $p(U \, F \, K \, I \, J \mid H) = p(U \, I \mid H) \, p(F \, K \, J \mid H)$ erfüllt ist,

wobei eine Äquivalenz besteht zwischen der 3. und der 4. Strichzählung, und wobei der angeführte Bedarfsfall genau dann eintritt, wenn URS(H) '-gekennzeichnete Elemente enthält, deren Berechnung die zum Fall H analogen Voraussetzungen erfordert.

5. Verfahren zur Ermittlung von Wahrscheinlichkeiten pathophysiologischer Zustände als Zwischenergebnisse der Diagnosefindung, dadurch gekennzeichnet, daß man mit den Bezeichnungen des Anspruchs 4 für ein beliebiges Ereignis H mit der unbekannten Zutreffenswahrscheinlichkeit $0 < p(H \mid H') < 1$ als Voraussetzungen fordert, daß

- die Ereignisse aus U, F, I, J mit der Wahrscheinlichkeit Null oder Eins vorliegen,
- (H K) sämtliche, diesem Verbund zuzuordnenden Ereignisse enthält,
- die Ereignisse in (H K) Selbständigkeitseigenschaft aufweisen,
- die Ereignisse in

$$\bigcup_{F_i \in F}(URS(F_i) \cup INH(F_i))$$

stochastisch unabhängig sind,

- $p(U \ F \ K \ I \ J \mid H) = p(U \ I \mid H) \ p(F \ K \ J \mid H)$ erfüllt ist,

wobei eine Äquivalenz besteht zwischen der 3. und der 4. Strichzählung.

6. Verfahren zur Ermittlung von Wahrscheinlichkeiten pathophysiologischer Zustände als Zwischenergebnisse der Diagnosefindung, dadurch gekennzeichnet, daß man mit den Bezeichnungen des Anspruchs 4 und den Voraussetzungen des Anspruchs 4 oder 5 für ein beliebiges Ereignis H mit der unbekannten Zutreffenswahrscheinlichkeit $0 < p(H \mid H') < 1$ die Beziehung

$$p(F \mid K_1 ... K_s JH) = \prod_{i=1}^{r} p(F_i \mid K_1 ... K_s JH)$$

verwendet, wobei exemplarisch $K := (K_1 ... K_s)$ gesetzt ist, daß man dann für alle i die Gleichung

$$p(F_i \mid K_1 ... K_s J H) = 1 - (1 - p(H.F_i \mid K_1 ... K_s J H)) \cdot \prod_{j=1}^{s}(1 - p(K_j.F_i \mid K_1 ... K_s JH))$$

nutzt, und daß man schließlich auf die darin enthaltenen Wahrscheinlichkeiten der Form $p(H.F_1 \mid K_1 ... K_s J H)$ bzw. $p(K_j.F_i \mid K_1 ... K_s J H)$ den A.L-Satz oder das A.L-Korollar 1 anwendet.

7. Verfahren zur Ermittlung von Wahrscheinlichkeiten pathophysiologischer Zustände als Zwischenergebnisse der Diagnosefindung, dadurch gekennzeichnet, daß man
1.
eine kausale Strukturierung zugrundelegt, die den in Anspruch 4 oder Anspruch 5 geforderten Voraussetzungen genügt, daß man
2.
für ein beliebiges, in der Kausalstruktur enthaltenes Ereignis $X_1$, dessen Zutreffenswahrscheinlichkeit $p(X_1 \mid X_1')$ unbekannt ist, eine sogenannte Wertungsgleichung $p(X_1 \mid X_1') = p(X_1 \mid W(X_1))$ aufstellt, wobei der Bedingungsverbund $W(X_1)$ unmittelbar mit $X_1$ verknüpfte und gegenüber $X_1$ stochastisch abhängige Ereignisse enthält, die negiert, unnegiert oder '-gekennzeichnet auftreten, daß man dies für alle m Ereignisse $X_1, ... , X_m$ mit unbekannter Zutreffenswahrscheinlichkeit ausführt, und daß man
3.
die Wertungsgleichungen in die Form gemäß Anspruch 1 bringt, daß man die erhaltenen bedingten Wahrscheinlichkeiten nach Möglichkeit linear gemäß Anspruch 2, nötigenfalls auch nicht-linear gemäß Anspruch 3 interpoliert, daß man
4.
eine Verkleinerung der bedingten Wahrscheinlichkeiten ausführt, zuächst bezüglich der vor dem Bedingungsstrich stehenden Ereignisse, unter Nutzung der Vollzähligkeit der beteiligten Ursachen und der Unabhängigkeit aller, auch der unbekannten Inhibitoren, daß man
5.
die bedingten Wahrscheinlichkeiten weiter verkleinert, jetzt bezüglich der Ereignisse nach dem Bedingungsstrich, unter Nutzung der für die beteiligten Ursachen eingeforderten Selbständigkeitseigenschaft, der Grundvoraussetzung für A.L-Satz und A.L-Korollar 1, daß man
6.
das solcherart aufgestellte Gleichungssystem von m Gleichungen in den m Unbekannten $p(X_1 \mid X_1'), ... , p(X_m \mid X_m')$ iterativ mit Hilfe des nicht-linearen Einzel- oder Gesamtschrittverfahrens, oder mit einem beliebigen anderen iterativen Verfahren löst.

Europäisches
Patentamt

**EUROPÄISCHER RECHERCHENBERICHT**

Nummer der Anmeldung

EP 99 10 2275

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG |
|---|---|---|---|
| X | EP 0 504 457 A (LIEBEL FRANZ PETER DR) 23. September 1992 (1992-09-23) | 1-4 | G06F19/00 G06F17/18 |
| A | * Seite 3, Zeile 1 - Seite 10, Zeile 25 * * Seite 22, Zeile 20 - Seite 24, Zeile 10 * --- | 5-7 | |
| A | EP 0 281 682 A (LIEBEL FRANZ PETER DR) 14. September 1988 (1988-09-14) * Seite 2, Zeile 1 - Zeile 24 * --- | 1-7 | |
| A | EP 0 399 082 A (LIEBEL FRANZ PETER DR) 28. November 1990 (1990-11-28) * Seite 2, Zeile 3 - Zeile 17 * --- | 1-7 | |
| A | EP 0 400 354 A (LIEBEL FRANZ PETER DR) 5. Dezember 1990 (1990-12-05) * Zusammenfassung * ----- | 1-7 | |

RECHERCHIERTE SACHGEBIETE

G06F

Der vorliegende Recherchenbericht wurde für alle Patentansprüche erstellt

| Recherchenort | Abschlußdatum der Recherche | Prüfer |
|---|---|---|
| DEN HAAG | 19. Juli 1999 | Schenkels, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 99 10 2275

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-07-1999

| Im Recherchenbericht angeführtes Patentdokument | | Datum der Veröffentlichung | Mitglied(er) der Patentfamilie | | Datum der Veröffentlichung |
|---|---|---|---|---|---|
| EP 0504457 | A | 23-09-1992 | KEINE | | |
| EP 0281682 | A | 14-09-1988 | DE | 3705964 A | 08-09-1988 |
| | | | DE | 3710559 A | 13-10-1988 |
| | | | DE | 3713692 A | 17-11-1988 |
| EP 0399082 | A | 28-11-1990 | EP | 0400354 A | 05-12-1990 |
| EP 0400354 | A | 05-12-1990 | EP | 0399082 A | 28-11-1990 |

EPO FORM P0461

Für nähere Einzelheiten zu diesem Anhang : siehe Amtsblatt des Europäischen Patentamts, Nr. 12/82